# EUROPEAN PATENT SPECIFICATION

(11) **EP 1 412 508 B1**
(45) Date of publication and mention of the grant of the patent: **26.04.2006**
(21) Application number: 02765335.1
(22) Date of filing: 01.08.2002
(51) Int. Cl.: C12N 15/85, C07K 14/16, C12Q 1/68

(54) **MOLECULAR VECTOR FOR THE GENOPHENOTYPIC CHARACTERISATION OF V3 SEQUENCES OF HIV-1 GP120**
VEKTOR ZUR PHÄNOTYPISCHEN CHARAKTERISIERUNG VON V3-SEQUENZE AUS DEM GP120-PROTEIN VON HIV-1
VECTEUR MOLECULAIRE DE CARACTERISATION GENOPHENOTYPIQUE DE SEQUENCES V3 DU VIH-1 GP120 ET METHODE DE PREPARATION CORRESPONDANTE

(30) Priority: 01.08.2001 IT RM20010468
(43) Date of publication of application: 28.04.2004
(73) Proprietor: CONSIGLIO NAZIONALE DELLE RICERCHE, 00185 Roma (IT)
(72) Inventor: CLEMENTI, Massimo, Università di Trieste, 34100 Trieste (IT); BAGNARELLI, Patrizia, Universita' di Ancona, 60020 Ancona (IT); MENZO, Stefano, Università di Ancona, 60020 Ancona (IT)
(74) Representative: Iannone, Carlo Luigi
(86) International application number: PCT/IT2002/000513
(87) International publication number: WO 2003/012148

(56) References cited:
- DE JONG J.J. ET AL.: "Human Immunodeficiency Virus Type 1 clones chimeric for the envelope V3 domain differ in syncytium formation and replication capacity." J. VIROL., vol. 66, no. 2, February 1992 (1992-02), pages 757-765, XP008013616
- HUNG C.-S. ET AL.: "Analysis of the critical domain in the V3 loop of human Immunodeficiency Virus Type 1 gp120 involved in CCR5 utilization." J. VIROL., vol. 73, no. 10, October 1999 (1999-10), pages 8216-8226, XP002230471

## Description

The present invention relates to a molecular vector for the genophenotypic characterisation of V3 sequences of HIV-1 gp120 and process for preparation thereof.

It is known that human immunodeficiency virus, HIV, presents on the outside envelope surface proteins, like gp120 and gp41, which play a key role during the initial phase of virus infection.

*Entry* phase of HIV virus in the host cell is a complex process developing in various steps, including gp120 surface glycoprotein binding to CD4 cell receptor of host cell, gp120 conformational modifications resulting in gp41 exposure and subsequent co-receptor selection before the starting of the fusion-type activity by gp41 glycoprotein. The latter, in fact, promotes the fusion of the lipid envelope and plasmatic membrane of the host cell to obtain a continuous unique membrane resulting in the introduction of the viral nucleoprotein core into cytoplasm of the host cell.

CD4 cell receptors are present on two types of hematic cells, namely CD4 lymphocytes (T helper lymphocytes) and monocytes. Various studies identified as co-receptors various surface molecules, named CCR5 and CXCR4, physiologically assigned to interact with some chemokines named RANTES (Regulated-Activations Normal T Expressed Secreted), MIP1-α, MIP1-β (Macrophage Inhibitory Protein) (Moore, J.P., A. Trkola and T. Dragic, 1997. Co-receptors for HIV-1 entry. Curr. Opin. Immunol. 9, 551-562). These co-receptors are both on T lymphocytes and macrophages.

gp120 variable sequences and particularly the 3 variable region (V3) play a key role during the very early steps of the HIV infection process, particularly for the binding and binding selection to the receptor and various co-receptors, respectively. Further V3 region is characterised by a remarkable inter and intra-host genomic diversity and is exposed to the host selection activities (Cocchi, F. et al., 1996, Nat. Med. 2, 1244-1247).

Accordingly it is known that during the natural history of HIV infection the viral population undergoes a non homogeneous genetic evolution resulting in viral variants (quasispecies) and characterising the disease progress.

During the genetic evolution the modifications involving the *env* structural gene are particularly important. The latter encodes for a polypeptide precursor, gp160, which subsequently is cleaved by viral proteases generating gp120 and gp41 glycoproteins.

Modifications of *env* gene and therefore V3 regions of gp120 affect the binding to the various co-receptors and develop in detectable parallelism with the disease progress.

Finally the HIV *entry* step, in addition to represent the main step of the viral replication to be overcome by main tests for active immunization, currently is also an attracting target for the development of new compounds with antiviral activity comprising both inhibitors of the bond between V3 region of HIV gp120 and CCR5 and CXCR4 co-receptors and of virus fusion with the host cell, respectively.

Due to these main reasons techniques and procedures suitable to study one or more aspects related to this step are important both as tools to investigate the infection from pathogenic point on view and potential diagnostic methodologies.

In the recent years individual molecular recombinant clones were developed to evaluate the functionality of partial deletion mutants of *env* gene sequences. In most cases evaluation studies of individual clones or chimeric viruses for research purposes were carried out. (Isaka, Y. et al., 1999, 264; 237-43; Chan, S. Y et al., J Virol. 73: 2350-8). For example, it is known (De Jong J.J. et al., J. Virol., vol. 66, no.2, February 1992, pages 757- 765) the construction of chimeric human immunodeficiency virus type 1 (HIV-1) molecular clones by means of a recombinant vector. This vector (pJJ25) consists of two components: a plasmid component able to carry out efficiently vector replication in bacteria and insert expression in eukaryotic cells and an HIV component characterised in that the V3 portion of the *env* gene has been deleted. In some cases procedures involving "complementation" of viral sequences present in two different subsequently co-transfected vectors, wherein one thereof carries the viral skeleton lacking for a sequence and the other carries the lacking sequence from heterologous derivation, were used; this approach, although interesting when used for research purposes, experiences inquiring and, at the end, inefficient when applied for diagnostic aspect.

From the above description it is apparent the need to have the availability of new tools and methodologies for the study of the structures and mechanisms involved in the *entry* step of the HIV infection.

The authors of the present invention therefore provided a method for the genophenotypic evaluation of genomic sequences and corresponding structures involved in the virus *entry* step of *in vivo* selected or *in vitro* generated variants.

Within the scope of the invention, therefore, a molecular vector to be advantageously used for the evaluation of the recombinant phenotype of the V3 region and corresponding analytical methodology were provided.

Molecular vector includes the HIV genome, modified by the deletion of the sequence encoding for V3 region and insertion of a new restriction site. This vector allows an easy insertion of exogenous V3 sequences and the production of recombinant virus (chimeric) after the transfection of the same vector in competent cells.

The inventive method allows easy and rapid cloning of V3 sequences in a molecular vector to be carried out and, after transfection, recombinant large scale viruses to be obtained and studied and, concurrently, the obtained clones to be handled easily by mutagenesis.

Said vector has been constructed and peculiar feature thereof is the availability for a routine work aiming at the evaluation of individual clones of the viral V3 sequence. It is possible by virtue of specific modifications carried out on the backbone. These modifications, designed in a such way not to modify the frameshift of the translation after the expression of the viral sequences occurring in the transfected cells, concern the region wherein the insertion for the cloning of the heterologous V3 sequence is carried out and the specific used restriction site.

A further advantage is represented by the easiness of the cloning improving the efficiency of a method suitable to a routine work.

The validation by means of reference strains has confirmed that the obtained recombinant virus maintains, in respect of the use of co-receptors, the characteristics of the strain from which the V3 sequence has been obtained.

An interesting feature for studies both about pathogenesis and resistance of drugs to inhibitors of co-receptor use is the possibility of obtaining mutants of V3 region in a simple, direct and rapid way and evaluating immediately phenotypic characteristics thereof.

It is therefore a specific object of the present invention a recombinant vector essentially consisting of: a) a plasmid component able to carry out exactly and efficiently vector replication in bacteria and viral insert expression in eukaryotic cells, respectively; b) an HIV component characterised in that the V3 portion sequence of *env* gene has been deleted and substituted by Nrul enzyme restriction site.

It is a further object of the present invention a method for genophenotypic evaluation of HIV genome sequences and corresponding structures involved in virus *entry* step of *in vivo* selected or *in vitro* generated variants, comprising essentially the following steps: 1) amplification of the sequence encoding for V3 region from a sample; 2) cloning of the amplification product in the inventive Nrul site of the vector in order to obtain a recombinant vector, 3) transfection of the recombinant vector in competent cells like, for example, 293T line cells, able to carry out HIV replication; 4) collection and purification of the obtained recombinant HIV virus; 5) infection of cell lines by obtained recombinant HIV viruses expressing different receptors and/or co-receptors able to bind HIV V3 sequence.

Preferably the amplification of the sequence encoding for V3 region from a sample is carried out by using following primers:

V3s 5'-TACAGCTGAAGTAATCTGTAGAAAT;

V3as 5'-TATTCCATTTTGCTCTACTAA.

Among receptors and co-receptors expressed by infectible cell lines there are CD4 receptors and CCR5 and CXCR4 co-receptors.

The present invention will be described now, by way of illustration but not limitation, according to preferred embodiments thereof, particularly with reference to the enclosed drawings, wherein:
Figure 1 shows the construction of the pNL-ΔV3 suitable to be used to obtain recombinant viruses for V3 sequence.
Figure 2 shows the procedure used in the study for the evaluation of the viral phenotype from V3 sequences obtained from clinical sample of infected patients.

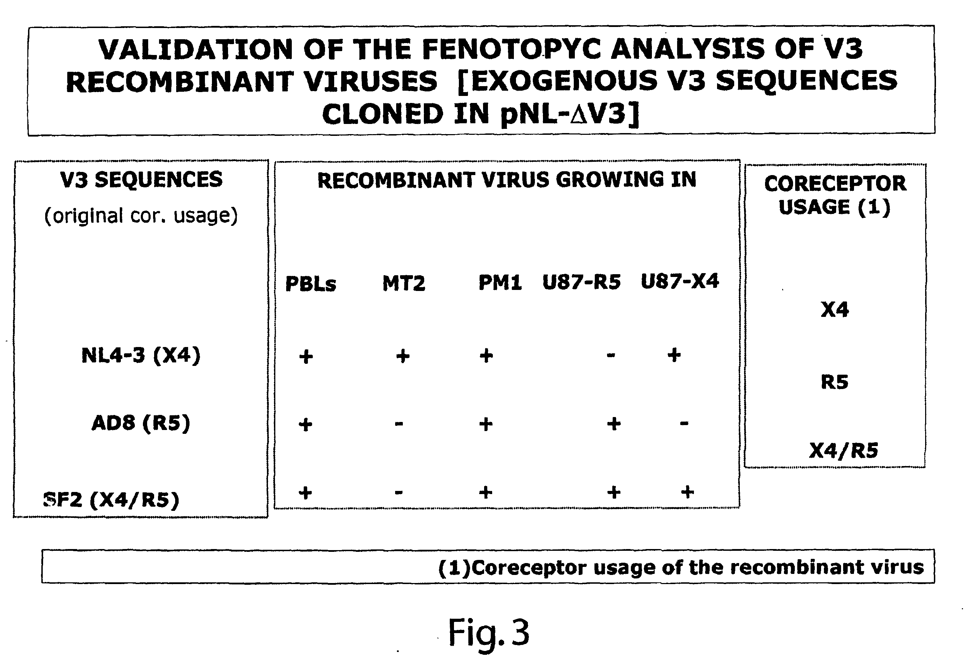

Figure 3 shows the results of the general validation of the procedure.

### Example 1

### Construction of pNL-ΔV3 vector

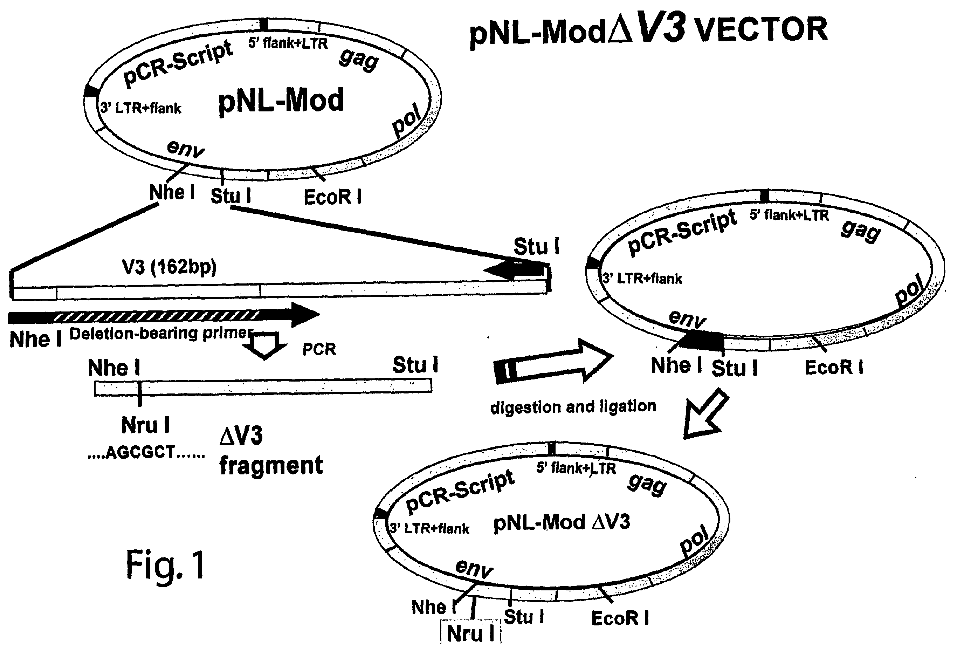

Vector suitable to obtain recombinant viruses of V3 sequences was obtained by using the procedure described in Figure 1.

HIV specific viral insert, contained in pNL4-3 vector (NIBSC-MCR Centralised Facility for AIDS Reagents; cat N. 2006), firstly was translated, by cutting and subsequent cloning, into pCR-Script plasmid (cat. Invitrogen). Successively the HIV *env* gene fragment limited by Nhel and Stul restriction sites and including V3 sequence was modified and amplified by PCR-directed mutagenesis.

Following two primers were used:

Nhel-Nrul primer
5'-GCgctagcTATCTGTTTTAAAGtcgcaTTATGGTTTTAGC;
[?]

StuI primer
5'-cctGTCCAAAGGTATCCTTTGA

First primer included a deletion of V3 sequence and, concurrently, generated a new restriction site of Nrul enzyme. The latter, whose sequence is AGCGCT, is not contained in the HIV genome and allows heterologous V3 sequences to be easily inserted.

The vector, named NLmodΔV3, was used for cloning of heterologous V3 sequences in Nrul site.

### Example 2

### Methodology for the study of phenotypic characteristics conferred by in vivo selected V3 sequences.

Molecular vector obtained according to example 1 was used in a methodology for the study of phenotypic viral characteristics conferred by *in vivo* selected V3 sequences from infected patients.

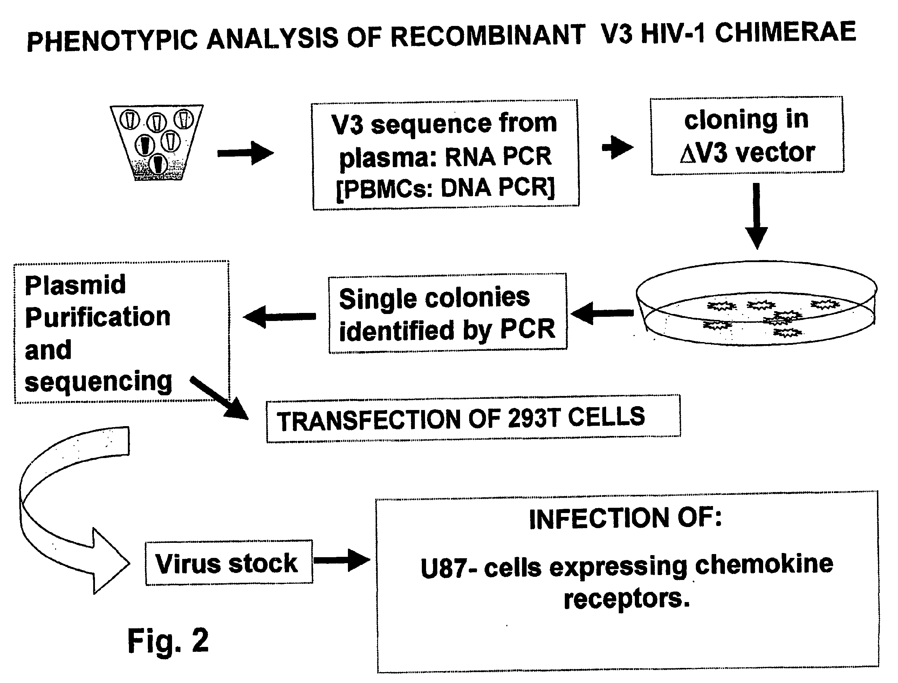

The methodology is showed in Figure 2 and is characterised by the following steps: amplification of V3 sequence from clinical sample using following primers:

V3s 5'-TACAGCTGAAGTAATCTGTAGAAAT;

V3as 5'-TATTCCATTTTTGCTCTACTAA.

Sequences are just outside V3 region and designed to allow cloning of the amplified in Nrul site.

Subsequent step is cloning of the amplification product within the new vector followed by transfection into competent cells, collection and purification of freshly produced recombinant virus (common backbone and clone specific V3 sequence), infection of different cell lines and positive CD4 cells expressing a single co-receptor.

Clonal V3 sequences, obtained by PCR carried out, alternatively, on plasma purified RNA or peripheral lymphocyte extracted DNA, were inserted into NLmodΔV3 vector by using Nrul restriction site. Successively competent bacterial cells were transformed and the plasmid was purified. Cloned V3 sequence was then amplified starting from the vector and sequenced. Complete vector was used to transfect 293T line cells (National Institue of Health - AIDS Research and Reference Reagent; cat N. 103) by lipofectine transfection. Infecting virus, produced from cells transfected by complete vector, was collected from cell supernatant and used to infect primary lymphocytes, MT2 cells (NIBSC - MCR Centralised Facility for AIDS Reagents; N. ARP014), PM1 cells (lymphoid T lines) and, particularly, U87 cells (NIBSC - MCR Centralised Facility for AIDS Reagents; N. ARP069-073) which express chemokine receptors. U87 cells are genetically modified in order to express individually anyone of HIV receptors used during the cell entry step.

Chemokine receptors, named CCR5 and CXCR4, are the most used by virus. HIV can be able to use CCR5 or CXCR4. There are viral strains able to use both receptors (dual-tropic strains).

It is apparent that the selection of binding to various co-receptors can *in vivo* affect the tropism, i.e. the ability of the virus to infect a specific cell or tissue, because receptors are differently distributed on the surface of infectible cells.

U87 cells which express individual receptors are therefore an excellent model to evaluate the viral phenotype with respect to the initial step of the infecting cycle. When it is used a viral skeleton on which the short V3 sequence only varies, the detected phenotypic modifications result from the sequence itself. In other words a biological model suitable to evaluate the significance of V3 sequence modifications will be available.

### Example 3

### Procedure validation

The procedure validation was carried out by cloning into the new vector V3 sequences of reference viral clones having known CCR5 and CXCR4 tropism and dual-tropic CCR5-CXCR4 clones. Particularly V3 sequences of viral NL4-3 clones were used, known as virus able to use AD8, CXCR4 receptor (Theodore Ts, et al., 1996, AIDS Res Hum Retrovir 12, 191-194) which uses dual-tropic CCR5 and SF2 (NIBSC-MCR Centralised Facility for AIDS Reagents; N ARP112.1) receptor, i.e. it is able to use either CCR5 or CXCR4 receptor.

Reported sequences were PCR amplified and cloned into NLmodΔV3 vector by using Nrul restriction site. According to the procedure described in figure 2 a complete extracellular virus was obtained, which was then used to infect various lymphoid lines and U87 cells which express CCR5 or CXCR4 on the surface. X4 or R5 terms mean virus able to use alternatively CXCR4 or CCR5 receptors.

Obtained results confirmed that recombinant viruses, differing for V3 region only, keep the original virus tropism.

Viral clones used for validation tests were produced by the authors (Menzo, S. et al., 1998, 12: 985-97; P. Bagnarelli et al., Journal of Virology 73, 3764-3777, 1999).

## Claims

1. Recombinant vector essentially consisting of: a) a plasmid component able to carry out exactly and efficiently vector replication in bacteria and viral insert expression in eukaryotic cells; b) an HIV component **characterised in that** the V3 portion sequence of *env* gene has been deleted and substituted by Nrul enzyme restriction site.

2. Method for genophenotypic evaluation of HIV genome sequences and corresponding structures involved during the virus *entry* step of *in vivo* or *in vitro* generated variants, comprising essentially the following steps: 1) amplification of the sequence encoding for V3 region from a sample; 2) cloning of the amplification product in the Nrul site of the vector of claim 1 in order to obtain a recombinant vector; 3) transfection of the recombinant vector in competent cells able to carry out HIV replication; 4) collection and purification of the obtained recombinant HIV virus; 5) infection of cell lines by obtained recombinant HIV viruses which express different receptors and/or co-receptors able to bind HIV V3 sequence.

3. Method according to claim 2 wherein the amplification of the sequence encoding for V3 region from a sample occurs by using the following primers:

V3s 5'-TACAGCTGAAGTAATCTGTAGAAAT;

V3as 5'-TATTCCATTTTGCTCTACTAA.

4. Method according to claim 2 wherein the receptors are CD4s and the co-receptors are CCR5s and CXCR4s.

5. Method according to claim 2 wherein the competent cells are 293T line cells.

## Revendications

1. Vecteur recombinant consistant essentiellement en : a) un composé plasmide capable de réaliser exactement et efficacement une réplication de vecteur dans une expression d'insertion bactérienne et virale dans des cellules eucaryotes ; b) un composé VIH **caractérisé en ce que** la portion de séquence V3 du gène *env* a été supprimée et substituée par un site de restriction de l'enzyme Nru1.

2. Procédé d'évaluation génophénotypique de séquences du génome du VIH et des structures correspondantes impliquées lors de l'étape d'entrée du virus de variants générés *in vivo* ou *in vitro,* comprenant essentiellement les étapes suivantes : 1) l'amplification de la séquence codant la région V3 d'un échantillon; 2) le clonage du produit d'amplification dans le site Nru1 du vecteur selon la revendication 1 afin d'obtenir un vecteur recombinant; 3) la transfection du vecteur recombinant dans des cellules compétentes capables de réaliser la réplication du VIH ; 4) la collecte et la purification du virus VIH recombinant obtenu; 5) l'infection de lignées cellulaires par les virus VIH recombinant obtenus qui expriment différents récepteurs et/ou co-récepteurs capables de lier la séquence VIH V3.

3. Procédé selon la revendication 2 dans lequel l'amplification de la séquence codant pour la région V3 d'un échantillon est effectuée en utilisant les amorces suivantes :

V3s 5'-TACAGCTGAAGTAATCTGTAGAAAT ;

V3as 5'-TATTCCATTTTGCTCTACTAA.

4. Procédé selon la revendication 2 dans lequel les récepteurs sont des récepteurs CD4 et les co-récepteurs sont des co-récepteurs CCR5 et des co-récepteurs CXCR4.

5. Procédé selon la revendication 2 dans lequel les cellules compétentes sont des cellules de lignée 293T.

## Patentansprüche

1. Rekombinater Vektor, im Wesentlichen bestehend aus a) einer Plasmidkomponente, die in der Lage ist, exakt und wirksam eine Vektorreplikation in Bakterien und eine Virus-Insert-Expression in eukariotischen Zellen durchzuführen; b) einer HIV-Komponente, die **dadurch gekennzeichnet ist, dass** die V3-Teil-Sequenz des *env-*Gens deletiert und durch die Restriktionsenzymstelle Nrul substituiert worden ist.

2. Verfahren zur geno-/phänotypischen Bewertung von HIV-Genomsequenzen und entsprechenden Strukturen, die am Virus-Eintrittschritt von *in vivo* oder *in vitro* erzeugten Varianten beteiligt sind, umfassend im Wesentlichen die folgenden Schritte: 1) Amplifikation der Sequenz, die die V3-Region kodiert, aus einer Probe; 2) Klonieren des Amplifikationsprodukts in die Nrul-Stelle des Vektors nach Anspruch 1, um einen rekonbinanten Vektor zu erhalten; 3) Transfektion des rekombinaten Vektors in kompetente Zellen, die zur Durchführung der HIV-Replikation in der Lage sind; 4) Sammeln und Reinigen des erhaltenen rekombinaten HIV-Virus; 5) Infektion von Zelllinien durch die erhaltenen rekombinanten Viren, die verschiedene Rezeptoren und/oder Corezeptoren exprimieren, die in der Lage sind, an die HIV-V3-Sequenz zu binden.

3. Verfahren nach Anspruch 2, wobei die Amplifikation der Sequenz, die die V3-Region aus einer Probe kodiert, unter Verwendung der folgenden Primer erfolgt:

V3s 5'-TACAGCTGAAGTAATCTGTAGAAAT;

V3as 5'-TATTCCATTTTGCTCTACTAA.

4. Verfahren nach Anspruch 2, wobei die Rezeptoren CD4s sind und die Corezeptoren CCR5s und CXCR4s sind.

5. Verfahren nach Anspruch 2, wobei die kompetenten Zellen Zellen der Line 293T sind.
